# EUROPÄISCHE PATENTSCHRIFT

(11) **EP 1 856 054 B1**
(45) Veröffentlichungstag und Bekanntmachung des Hinweises auf die Patenterteilung: **17.09.2008**
(21) Anmeldenummer: 06707100.1
(22) Anmeldetag: 21.02.2006
(51) Int. Cl.: C07D 231/14, A01N 43/56

(54) **PYRAZOLYLCARBOXANILIDE**
PYRAZOLYLCARBOXANILIDES
PYRAZOLYLCARBOXANILIDES

(30) Priorität: 02.03.2005 DE 102005009458
(43) Veröffentlichungstag der Anmeldung: 21.11.2007
(73) Patentinhaber: Bayer CropScience AG, 40789 Monheim (DE)
(72) Erfinder: DUNKEL, Ralf, F-69001 Lyon (FR); ELBE, Hans-ludwig, 42329 Wuppertal (DE); GREUL, Jörg Nico, 42799 Leichlingen (DE); GAYER, Herbert, 40789 Monheim Am Rhein (DE); SEITZ, Thomas, 40764 Langenfeld (DE); DAHMEN, Peter, 41470 Neuss (DE); WACHENDORFF-NEUMANN, Ulrike, 56566 Neuwied (DE)
(86) Internationale Anmeldenummer: PCT/EP2006/001520
(87) Internationale Veröffentlichungsnummer: WO 2006/092213

(56) Entgegenhaltungen:
- WO-A-97/08148
- WO-A-03/070705

## Beschreibung

Die vorliegende Erfindung betrifft neue Pyrazolylcarboxanilide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vgl. z.B. WO 03/070705, EP 0 545 099 und JP 9132567). Die Wirksamkeit der dort beschriebenen Stoffe ist gut, lässt aber bei niedrigen Aufwandmengen in manchen Fallen zu wünschen übrig.

Es wurden nun neue Pyrazolylcarboxanilide der Formel (I) 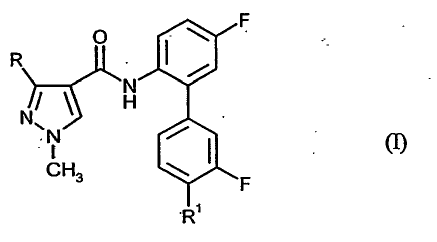 gefunden, in welcher
- R: für Difluormethyl oder Trifluormethyl steht und
- R¹: für Chlor oder Methyl steht.

Weiterhin wurde gefunden, dass man Pyrazolylcarboxanilide der Formel (I) erhält, indem man
a) Pyrazolylcarbonsäurehalogenide der Formel (II) 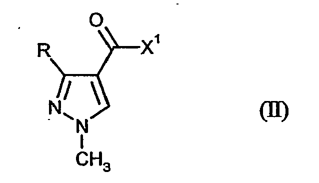 in welcher
   - R: die oben angegebenen Bedeutungen hat und
   - X¹: für Halogen steht,
   mit Anilinderivaten der Formel (III) 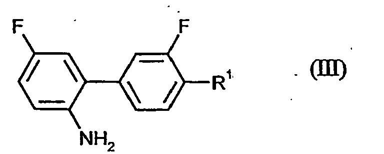 in welcher R¹ die oben angegebenen Bedeutungen hat,
   gegebenenfalls in Gegenwart eines Säurebindemittels und gegebenenfalls in Gegenwart eines Verdünnungsmittels umsetzt, oder
b) Halogenpyrazolylcarboxanilide der Formel (IV) 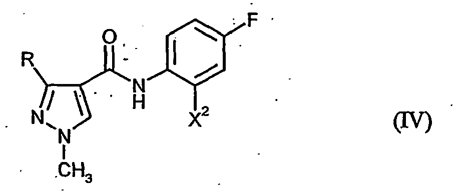 in welcher
   - R: die oben angegebenen Bedeutungen hat und
   - X²: für Brom oder Iod steht,
   mit Boronsäurederivaten der Formel (V) 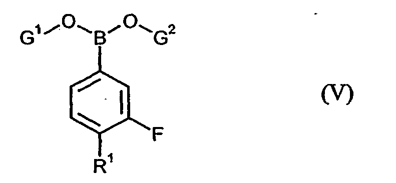 in welcher
   - R¹: die oben angegebenen Bedeutungen hat und
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Halogenpyrawlylcarboxanilide der Formel (IV) 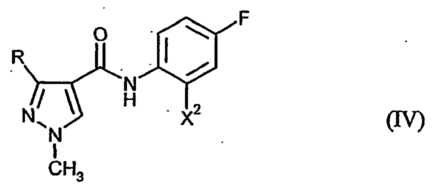 in welcher
   - R: die oben angegebenen Bedeutungen hat und
   - X²: für Brom oder Iod steht,
   in einer ersten Stufe mit einem Diboran-Derivat der Formel (VI) 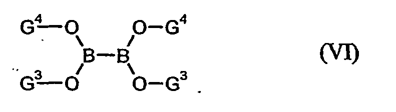 in welcher
   G³ und G⁴ jeweils für Alkyl oder gemeinsam für Alkandiyl stehen,
   in Gegenwart eines Katalysators, gegebenenfalls in Gegenwart eines Säurebindemittels und gegebenenfalls in Gegenwart eines Verdünnungsmittels umsetzt und ohne Aufarbeitung in einer zweiten Stufe mit Halogenbenzolderivaten der Formel (VII) 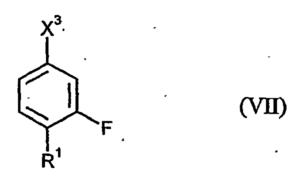 in welcher
   - R¹: die oben angegebenen Bedeutungen hat und
   - X³: für Brom, Iod oder Trifluormethylsulfonyloxy steht,
   in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die neuen Pyrazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Pyrazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Pyrazolylcarboxanilide sind durch die Formel (I) allgemein definiert. Formel (I) fasst folgende vier Pyrazolylcarboxanilide zusammen:
N-(4'-Chlor-3',5-difluorbiphenyl-2-yl)-3-(difluormethyl)-1-methyl-1H-pyrazol-4-carboxamid
N-(4'-Chlor-3',5-difluorbiphenyl-2-yl)-1-methyl-3-(trifluormethyl)-1H-pyrazol-4-carboxamid
N-(3',5-Difluor-4'-methylbiphenyl-2-yl)-1-methyl-3-(difluormethyl)-1H-pyrazol-4-carboxamid
N-(3',5-Difluor-4'-methylbiphenyl-2-yl)-1-methyl-3-(trifluormethyl)-1H-pyrazol-4-carboxamid

Verwendet man beispielsweise 1-Methyl-3-(trifluormethyl)-1H-pyrazol-4-carbonylchlorid und 4'-Chlor-3',5-difluor-1,1'-biphenyl-2-amin als Ausgangsstoffe sowie ein Base, so kann der Verlauf des erfindungsgemäßen Verfahrens a) durch folgende Reaktionsgleichung veranschaulicht werden:

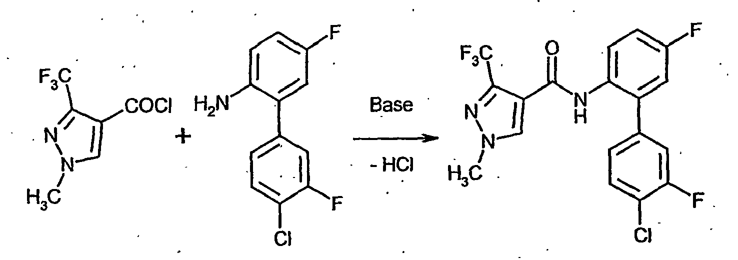

Die zur Durchführung des erfindungsgemäßen Verfahrens a) als Ausgangsstoffe benötigten Pyrazolylcarbonsäurehalogenide sind durch die Formel (II) allgemein definiert. In dieser Formel (II) steht R für Difluormethyl oder Trifluormethyl. X¹ steht bevorzugt für Chlor.

Die Pyrazolylcarbonsäurehalogenide der Formel (II) sind bekannt und/oder lassen sich nach bekannten Verfahren herstellen (vgl. z.B. JP 01290662 und US 5,093,347).

Die weiterhin zur Durchführung des erfindungsgemäßen Verfahrens a) als Ausgangsstoffe benötigten Anilin-Derivate sind durch die Formel (III) allgemein definiert. In dieser Formel (III) steht R¹ für Chlor oder Methyl.

Die Anilin-Derivate der Formel (III) sind bekannt und/oder lassen sich nach bekannten Verfahren herstellen (vgl. WO 03/070705).

Verwendet man *N*-(2-Brom-4-fluorphenyl)-1-methyl-3-(trifluormethyl)-1H-pyrazol-4-carboxamid und 4-Chlor-3-fluorphenylboronsäure als Ausgangsstoffe sowie einen Katalysator und eine Base, so kann der Verlauf des erfindungsgemäßen Verfahrens b) durch folgende Reaktionsgleichung veranschaulicht werden:

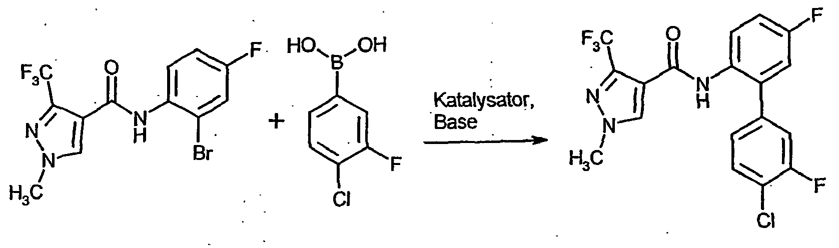

Die zur Durchführung des erfindungsgemäßen Verfahrens b) als Ausgangsstoffe benötigten Halogenpyrazolylcarboxanilide sind durch die Formel (IV) allgemein definiert. In dieser Formel (IV) steht R für Difluormethyl oder Trifluormethyl. X² steht bevorzugt für Brom oder Iod.

Die Halogenpyrazolylcarboxanilide der Formel (IV) sind bekannt (vgl. WO 03/070705).

Die weiterhin zur Durchführung des erfmdungsgemäßen Verfahrens b) als Ausgangsstoffe benötigten Boronsäurederivate sind durch die Formel (V) allgemein definiert. In dieser Formel (V) steht R¹ für Chlor oder Methyl. G¹ und G² stehen bevorzugt jeweils für Wasserstoff oder zusammen für Tetramethylethylen.

Boronsäuren der Formel (V) sind bekannte Synthesechemikalien. Sie können auch unmittelbar vor der Reaktion direkt aus Halogenbenzolderivaten und Boronsäureestern hergestellt und ohne Aufarbeitung weiter umgesetzt werden.

Verwendet man beispielsweise *N*-(2-Brom-4-fluorphenyl)-1-methyl-3-(trifluormethyl)-1H-pyrazol-4-carboxamid' und 4,4,4',4',5,5,5',5'-Octamethyl-2,2'-bi-1,3,2-dioxaborolan in der ersten Stufe und weiterhin 5-Brom-2-chlor-1-fluorbenzol in der zweiten Stufe als Ausgangsstoffe, sowie in jeder Stufe einen Katalysator und eine Base, so kann der Verlauf des erfindungsgemäßen Verfahrens c) durch folgende Reaktionsgleichung veranschaulicht werden:

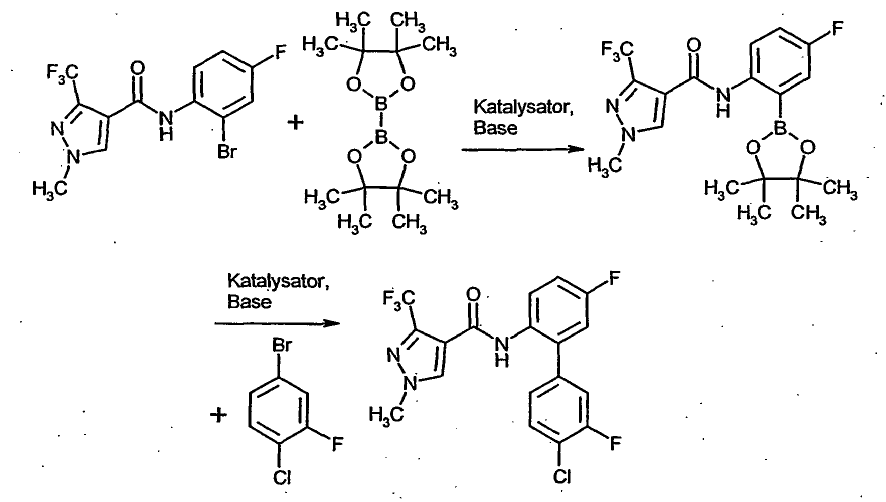

Die zur Durchführung des erfindungsgemäßen Verfahrens c) als Ausgangsstoffe benötigten Halogenpyrazolylcarboxanilide der Formel (IV) sind bereits weiter oben im Zusammenhang mit dem erfindungsgemäßen Verfahren b) beschrieben worden.

Die zur Durchführung des erfindungsgemäßen Verfahrens c) weiterhin als Ausgangsstoffe benötigten Diboran-Derivate sind durch die Formel (VI) allgemein definiert. In dieser Formel (VI) stehen G³ und G⁴ bevorzugt jeweils für Methyl, Ethyl, Propyl, Butyl oder gemeinsam für Tetramethylethylen.

Die Diboran-Derivate der Formel (VI) sind allgemein bekannte Synthesechemikalien.

Die außerdem zur Durchführung des erfindungsgemäßen Verfahrens c) als Ausgangsstoffe benötigten Halogenbenzolderivate sind durch die Formel (VII) allgemein definiert. In dieser Formel (VII) steht R¹ für Chlor oder Methyl. X³ steht für bevorzugt für Brom, Iod oder Trifluormethylsulfonyloxy.

Die Halogenbenzolderivate der Formel (VII) sind allgemein bekannte Synthesechemikalien.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall oder Alkalimetallhychide, -hydroxide, -amide, -alkoholate, -acetate, -carbonate oder -hydrogencarbonate, wie z.B. Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im Allgemeinen arbeitet man bei Temperaturen von 0°C bis 150°C, vorzugsweise bei Temperaturen von 20°C bis 110°C.

Zur Durchführung des erfindungsgemäßen Verfahrens a) zur Herstellung der Verbindungen der Formel (I) setzt man pro Mol des Pyrazolylcarbonsäurehalogenides der Formel (II) im Allgemeinen 0,2 bis 5 Mol, vorzugsweise 0,5 bis 2 Mol an Anilinderivat der Formel (III) ein.

Als Verdünnungsmittel zur Durchführung der erfindungsgemäßen Verfahren b) und c) kommen alle inerten organischen Lösungsmittel in Betracht Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, s-oder t-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens b) und c) in einem größeren Bereich variiert werden. Im Allgemeinen arbeitet man bei Temperaturen von 0°C bis 150°C, vorzugsweise bei Temperaturen von 20°C bis 110°C.

Die erfindungsgemäßen Verfahren b) und c) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -fluoride, -phosphate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Kaliumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b) und c) werden in Gegenwart eines Katalysators, wie z.B. eines Palladiumsalzes oder -komplexes, durchgeführt. Hierzu kommen vorzugsweise Palladiumchlorid, Palladiumacetat, Tetrakis-(triphenylphosphin)-palladium, Bis-(triphenylphosphin)-palldiumdichlorid oder 1,1'-Bis(diphenylphosphino)ferrocenpalladium(II)chlorid infrage.

Es kann auch ein Palladiumkomplex in der Reaktionsmischung erzeugt werden, wenn man ein Palladiumsalz und einen Komplexligand, wie beispielsweise Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium-3-(diphenylphosphino)benzolsulfonat, Tris-2-(methoxyphenyl)-phosphan, 2,2'-Bis-(diphenylphosphan)-1,1'-binaphthyl, 1,4-Bis(diphenylphosphan)-butan, 1,2-Bis-(diphenyl-phosphan)-ethan, 1,4-Bis(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens b) zur Herstellung der Verbindungen der Formel (I) setzt man pro Mol des Halogenpyrazolylcarboxanilids der Formel (IV) im Allgemeinen 1 bis 15 Mol, vorzugsweise 1 bis 5 Mol an Boronsäurederivat der Formel (V) ein.

Zur Durchführung des erfindungsgemäßen Verfahrens c) zur Herstellung der Verbindungen der Formel (I) setzt man pro Mol des Halogenpyrazolylcarboxanilids der Formel (IV) im Allgemeinen 1 bis 15 Mol, vorzugsweise 1 bis 5 Mol an Diboran-Derivat der Formel (VI) und 1 bis 15 Mol, vorzugsweise 1 bis 5 Mol an Halogenbenzolderivat der Formel (VII) ein.

Die erfindungsgemäßen Verfahren a), b) und c) werden im Allgemeinen unter Normaldruck durchgeführt. Es ist jedoch auch möglich, unter erhöhtem oder vermindertem Druck - im Allgemeinen zwischen 0, 1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
   Blumeria-Arten, wie beispielsweise Blumeria graminis;
   Podosphaera-Arten, wie beispielsweise Podosphaera leucotricha;
   Sphaerotheca-Arten, wie beispielsweise Sphaerotheca fuliginea;
   Uncinula-Arten, wie beispielsweise Uncinula necator;
Erkrankungen, hervorgerufen durch Erreger von Rostkrankheiten wie z.B.
   Gymnosporangium-Arten, wie beispielsweise Gymnosporangium sabinae
   Hemileia-Arten, wie beispielsweise Hemileia vastatrix;
   Phakopsora-Arten, wie beispielsweise Phakopsora pachyrhizi und Phakopsora meibomiae;
   Puccinia-Arten, wie beispielsweise Puccinia recondita;
   Uromyces-Arten, wie beispielsweise Uromyces appendiculatus;
Erkrankungen, hervorgerufen durch Erreger der Gruppe der Oomyceten wie z.B.
   Bremia-Arten, wie beispielsweise Bremia lactucae;
   Peronospora-Arten, wie beispielsweise Peronospora pisi oder P. brassicae;
   Phytophthora-Arten, wie beispielsweise Phytophthora infestans;
   Plasmopara-Arten, wie beispielsweise Plasmoparaviticola;
   Pseudoperonospora-Arten, wie beispielsweise Pseudoperonospora humuli oder Pseudoperonospora cubensis;
   Pythium-Arten, wie beispielsweise Pythium ultimum;
Blattfleckenkrankheiten und Blattwelken, hervorgerufen durch z.B.
   Alternaria-Arten, wie beispielsweise Alternaria solani;
   Cercospora-Arten, wie beispielsweise Cercospora beticola;
   Cladiosporum-Arten, wie beispielsweise Cladiosporium cucumerinum;
   Cochliobolus-Arten, wie beispielsweise Cochliobolus sativus
   (Konidienform: Drechslera, Syn: Helminthosporium);
   Colletotrichurn-Arten, wie beispielsweise Colletotrichum lindemuthanium;
   Cycloconium-Arten, wie beispielsweise Cycloconium oleaginum;
   Diaporthe-Arten, wie beispielsweise Diaporthe citri;
   Elsinoe-Arten, wie beispielsweise Elsinoe fawcettii;
   Gloeosporium-Arten, wie beispielsweise Gloeosporium laeticolor;
   Glomerella-Arten, wie beispielsweise Glomerella cingulata;
   Guignardia-Arten, wie beispielsweise Guignardia bidwelli;
   Leptosphaeria-Arten, wie beispielsweise Leptosphaeria maculans;
   Magnaporthe-Arten, wie beispielsweise Magnaporthe grisea;
   Mycosphaerella-Arten, wie beispielsweise Mycosphaerelle graminicola;
   Phaeosphaeria-Arten, wie beispielsweise Phaeosphaeria nodorum;
   Pyrenophora-Arten, wie beispielsweise Pyrenophora teres;
   Ramularia-Arten, wie beispielsweise Ramularia collo-cygni;
   Rhynchosporium-Arten, wie beispielsweise Rhynchosporium secalis;
   Septoris-Arten, wie beispielsweise Septoria apii;
   Typhula-Arten, wie beispielsweise Typhula incarnata;
   Venturia-Arten, wie beispielsweise Venturia inaequalis;
Wurzel- und Stengelkrankheiten, hervorgerufen durch z.B.
   Corticium-Arten, wie beispielsweise Corticium graminearum;
   Fusarium-Arten, wie beispielsweise Fusarium oxysporum;
   Gaeumannomyces-Arten, wie beispielsweise Gaeumannomyces graminis;
   Rhizoctonia-Arten, wie beispielsweise Rhizoctonia solani;
   Tapesia-Arten, wie beispielsweise Tapesia acuformis;
   Thielaviopsis-Arten, wie beispielsweise Thielaviopsis basicola;
Ähren- und Rispenerkrankungen (inklusive Maiskolben), hervorgerufen durch z.B.
   Alternaria-Arten, wie beispielsweise Alternaria spp.;
   Aspergillus-Arten, wie beispielsweise Aspergillus flavus;
   Cladosporium-Arten, wie beispielsweise Cladosporium spp.;
   Claviceps-Arten, wie beispielsweise Claviceps purpurea;
   Fusarium-Arten, wie beispielsweise Fusarium culmorum;
   Gibberella-Arten, wie beispielsweise Gibberella zeae;
   Monographella-Arten, wie beispielsweise Monographella nivalis;
Erkrankungen, hervorgerufen durch Brandpilze wie z.B.
   Sphacelotheca-Arten, wie beispielsweise Sphacelotheca reiliana;
   Tilletia-Arten, wie beispielsweise Tilletia caries;
   Urocystis-Arten, wie beispielsweise Urocystis occulta;
   Ustilago-Arten, wie beispielsweise Ustilago nuda;
Fruchtfäule hervorgerufen durch z.B.
   Aspergillus-Arten, wie beispielsweise Aspergillus flavus;
   Botrytis-Arten, wie beispielsweise Botrytis cinerea;
   Penicillium-Arten, wie beispielsweise Penicillium expansum;
   Sclerotinia-Arten, wie beispielsweise Sclerotinia sclerotiorum;
   Verticilium-Arten, wie beispielsweise Verticilium alboatrum;
Samen- und bodenbürtige Fäulen und Welken, sowie Sämlingserkrankungen, hervorgerufen durch z.B.
   Fusarium-Arten, wie beispielsweise Fusarium culmorum;
   Phytophthora Arten, wie beispielsweise Phytophthora cactorum;
   Pythium-Arten, wie beispielsweise Pythium ultimum;
   Rhizoctonia-Arten, wie beispielsweise Rhizoctonia solani;
   Sclerotium-Arten, wie beispielsweise Scierotium rolfsii;
Krebserkrankungen, Gallen und Hexenbesen, hervorgerufen durch z.B.
   Nectria-Arten, wie beispielsweise Nectria galligena;
Welkeerkrankungen hervorgerufen durch z.B.
   Monilinia-Arten, wie beispielsweise Monilinia laxa;
Deformationen von Blättern, Blüten und Früchten, hervorgerufen durch z.B.
   Taphrina-Arten, wie beispielsweise Taphrina deformans;
Degenerationserkrankungen holziger pflanzen, hervorgerufen durch z.B.
   Esca-Arten, wie beispielsweise Phaemoniella clamydospora;
   Blüten- und Samenerkrankungen, hervorgerufen durch z.B.
   Botrytis-Arten, wie beispielsweise Botrytis cinerea;
Erkrankungen von Pflanzenknollen, hervorgerufen durch z.B.
   Rhizoctonia-Arten, wie beispielsweise Rhizoctonia solani;
Erkrankungen, hervorgerufen durch bakterielle Erreger wie z.B.
   Xanthomonas-Arten, wie beispielsweise Xanthomonas campestris pv. oryzae;
   Pseudomonas-Arten, wie beispielsweise Pseudomonas syringae pv. lachrymans;
   Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B.
   Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllosticä Leaf Spot (Phyllosticta sojaecola), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycins), Stemphylium Leaf Blight (Stemphylium botryosum), Target Spot (Corynespora cassiicola)
Pilzkrankheiten an Wurzeln und der Stängelbasis verursacht durch z.B.
   Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilen, Leder, Holz, Anstrichmittel und Kunststoffartikel, Kühlschmierstoffe und andere Materialien sein, die von Mikro-organismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektizide verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Diethofencarb, Ethaboxam, Fuberidazole, Pencycuron, Thiabendazole, Thiophanate-methyl, Zoxamide;
3) Inhibitoren der Respiration (Atmungsketten-Inhibitoren):
   3.1) Inhibitoren am Komplex I der Atmungskette: z.B. Diflumetorim;
   3.2) Inhibitoren am Komplex II der Atmungskette: z.B. Boscalid/Nicobifen, Carboxin, Fenfuram, Flutolanil, Furametpyr, Furmecyclox, Mepronil, Oxycarboxin, Penthiopyrad, Thifluzamide;
   3.3) Inhibitoren am Komplex III der Atmungskette: z.B. Amisulbrom, Azoxystrobin, Cyazofamid, Dimoxystrobin, Enestrobin, Famoxadone, Fenamidone, Fluoxastrobin, Kresoxim-methyl, Metominostrobin, Orysastrobin, Picoxystrobin, Pyraclostrobin, Trifloxystrobin;
4) Entkoppler: z.B. Dinocap, Fluazinam, Meptyldinocap;
5) Inhibitoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bitertanol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole;
10) Inhibitoren der Zellwandsynthese: z.B. Benthiavalicarb, Dimethomorph, Flumorph, Iprovalicarb, Polyoxins, Polyoxorim, Validamycin A;
11) Inhibitoren der Melanin-Biosynthese: z.B. Carpropamid, Diclocymet, Fenoxanil, Phthalide, Pyroquilon, Tricylazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schweferlzubereitungen wie z.B. Calcium polysulphide, Thiram, Tolylfluanid, Zineb, Ziram;
14) eine Verbindung aus der folgenden Liste: (2E)-2-(2-{[6-(3-Chlor-2-methylphenoxy)-5-fluorpyrimi-din-4-yl]oxy}phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2(metheoxyimino)-N-methylacetamid, 1-(4-Chlorphenyl)-2-(1H-1,2,4-triazol-1-yl)cycloheptanol, 1-[(4-Methoxyphenoxy)methyl]-2,2-dimethylpropyl-1H-imidazol-1-carboxylat, 2-(4-Chlorphenyl)-N-{2-[3-methoxy-4-(prop-2-yn-1-yloxy)phenyl]ethyl}-2-(prop-2-yn-1-yloxy)acetamid, 2,3,5,6-Tetrachlor-4-(methylsulfonyl)pyridin, 2-Butoxy-6-iod-3-propyl-4H-chromen-4-on, 2-Chlor-N-(1,1,3-trimethyl-2,3-dihydro-1H-inden-4-yl)nicotinamid, 2-Phenylphenol und Salze davon, 3,4,5-Trichlorpyridin-2,6-dicarbonitril, 3,4-Dichlor-N-(2-cyanophe-nyl)isothiazol-5-carboxamid, 3-[5-(4-Chlorphenyl)-2,3-dimethylisoxazolidin-3-yl]pyridin, 5-Chlor-6-. (2,4,6-trifluorophenyl)-N-[(1R)-1,2,2-trimethylpropyl][1,2,4]triazolo[1,5-a]pyrimidin-7-amin, 5-Chlor-7-(4-methylpiperidin-1-yl)-6-(2,4,6-trifluorphenyl)[1,2,4]triazolo[1,5-a]pyrimidin, 5-Chlor-N-[(1R)-1,2-dimethylpropyl]-6-(2,4,6-trifluorphenyl)[1,2,4]triazolo[1,5-a]pyrimidin-7-amin, 8-Hydroxyquinolinsulfat, Benthiazole, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulphate, Diphenylamine, Ferimzone, Flumetover, Fluopicolide, Fluoroimide, Flusulfamide, Fosetyl-Aluminium, Fosetyl-Calcium, Fosetyl-Sodium, Hexachlorobenzene, Irumamycin, Methasulfocarb, Methyl (2-chlor-5-{(1E)N-[(6-methylpyridin-2-yl)methoxy]ethanimidoyl}benzyl)carbamat, Methyl (2E)-2-{2-[({cyclopropyl[(4-methoxyphenyl)imino]methyl}thio)methyl]phenyl}-3-methoxy-acrylat, Methyl 1-(2,2-dimethyl-2,3-dihydro-1H-inden-1-yl)-1H-imidazol-5-carboxylat, Methyl 3-(4-chlorphenyl)-3-{[N-(isopropoxycarbonyl)valyl]amino}propanoat, Methyl isothiocyanate, Metrafenone, Mildiomycin, N-(3',4'-Dichlor-5-fluorbiphenyl-2-yl)-3-(difluormethyl)-1-methyl-1H-pyrazol-4-carboxamid, N-(3-Ethyl-3,5,5-trimethylcyclohexyl)-3-(formylamino)~2-hydroxybenzamid, N-(4-Chlor-2-nitrophenyl)N-ethyl-4-methylbenzensulfonamid, N-[(5-Brom-3-chlorpyridin-2-yl)methyl]-2,4-dichlornicotinamid, N-[1-(5-Brom-3-chlorpyridin-2-yl)ethyl]-2,4-dichlornicotinamid, N-[1-(5-Brom-3-chlorpyridin-2-yl)ethyl]-2-fluor-4-iodnicotinamid, N-[2-(4-{[3-(4-Chlorphenyl)prop-2-yn-1-yl]oxy}-3-methoxyphenyl)ethyl]-N²-(methylsulfonyl)valinamid, N-{(Z)-[(Cyclopropylmethoxy)imino][6-(di-fluormethoxy)-2,3-difluorphenyl]methyl}-2-phenylacetamid, N-{2-[3-Chlor-5-(trifuormethyl)pyridin 2-yl]ethyl}-2-(trifluormethyl)benzamid, Natamycin, Nickel Dimethyldithiocarbamate, Nitrothalisopropyl, O-{1-[(4-Methoxyphenoxy)methyl]-2,2-dimethylpropyl} 1H-Imidazol-1-carbothioat, Octhilinone, Oxamocarb, Oxyfenthiin, Pentachlorophenol und Salze, Phosphorsäure und ihre Salze, Piperalin, Propamocarb Fosetylate, Propanosine-Sodium, Proquinazid, Pyrrolnitrine, Quintozene, Tecloftalam, Tecnazene, Triazoxide, Trichlamide, Zarilamid.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvös/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoäte, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (1R-isomer), Esfenvalerate, Etofenprox, Fenfluthrin, Fenpropathrin, Fenpyrithrin, Fenvalerat, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer); Tralomethrin, transfluthrin, ZXI 8901, Pyrethrins (pyrethrum))
   2.2 Oxadiazine (z.B. Indoxacarb)
*3. Acetylcholin-Rezeptor-Agonisten*/*-Antragonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avermectin, Emamectin, Emamectin-benzoate, ivermectin, Milbemectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Seite-I-Elektronentransportinhibitoren*
   12.1 METTs (z.B. Fenazaquin, Fenpyroximate, Pyrimidifen, Pyridaben, Tebufenpyrad, Tolfenpyrad)
   12.2 Hydramethylnone
   12.3 Dicofol
*13. Seite-II-Elektronentransportinhibitoren*
   13.1-Rotenone
*14. Seite-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Phthalamide*
   (z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechen-de 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmenge an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeich-nungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

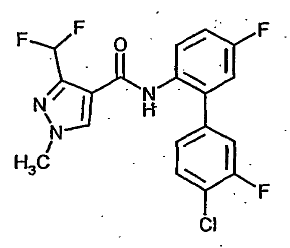

Zu einer Mischung bestehend aus 2,96 g (16,8 mmol) 3-Difluormethyl-1-methyl-1H-pyrazol-4-carbonsäure in 100 ml Dichlormethan, werden 1,6 ml (18,4 mmol) Oxalsäuredichlorid und 0,2 ml Dimethylformamid gegeben. Nach 2 Stunden bei Raumtemperatur wird eine Lösung bestehend aus 3,83 g (16,0 mmol) 4'-Chlor-5,3'-difluorbiphenyl-2-yl-amin und 2,9 ml (20,8 mmol) in 100 ml Dichlormethan, zugegeben. Das Reaktionsgemisch wird für 16 Stunden bei Raumtemperatur gerührt. Zur Aufarbeitung wird das Reaktionsgemisch auf Wasser gegeben, die organische Phase abgetrennt, über Magnesiumsulfat getrocknet und im Vakuum aufkonzentriert. Säulenchromatographie (Petrolether / Aceton 3:1) liefert 5,94 g (93 % der Theorie) N-(4'-Chlor-3,5-difluorbiphenyl-2-yl)-3-(difluormethyl)-1-methyl-1H-pyrazol-4-carboxamid mit dem logP (pH 2,3) = 3,05.

Analog Beispiel 1 und gemäß den allgemeinen Versuchsvorschriften in der Beschreibung erhält man folgende Verbindungen:
3-Difluormethyl-1-methyl-1*H*-pyrazol-4-carbonsäure (5,3'-difluor-4'-methyl-biphenyl-2-yl)-amid logP (pH 2,3) = 3,05
1-Methyl-3-trifluormethyl-1*H*-pyrazol-4-carbonsäure (5,3'-difluor-4'-methyl-biphenyl-2-yl)-amid logP (pH 2,3) = 3,27
1-Methyl-3-trifluormethyl-1*H*-pyrazol-4-carbonsäure (4'-chlor-5,3'-difluor-biphenyl-2-yl)-amid logP (pH 2,3) = 3,26

### Herstellung von Ausgangsstoffen der Formel (III)

### Beispiel (III-1)

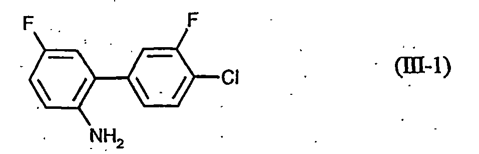

Unter Schutzgasatmosphäre wird ein Gemisch bestehend aus 30,0 g (0,17 mol) 4-Chlor-3-fluor-phenyl-boronsäure und 29,7 g (0,16 mol) 2-Brom-4-fluoranilin in 170 ml Toluol und 17 ml Ethanol mit 3,6 g (0,003 mol) Tetrakis(triphenylphosphin)palladium (0) versetzt und für 16 h bei 80°C gerührt. Nach Zugabe von 200 ml Toluol und 200 ml Wasser, wird die Organische Phase abgetrennt, über Magnesiumsulfat getrocknet und im Vakuum aufkonzentriert. Säulenchromatographie (Petrolether / Aceton 4:1) lie-fert 26,1 g (70 % der Theorie) 4'-Chlor-5,3'-difluorbiphenyl-2-yl-amin mit dem logP (pH 2,3) =3,18.

### Beispiel (III-2)

Ausgehend von 4-Methyl-3-fluor-phenylboronsäure und 2-Brom-4-fluoranilin erhält man analog Beispiel (III-1) die Verbindung 5,3'-Difluoro-4'-methyl-biphenyl-2-ylamin mit dem logP (pH 2,3) = 2,94.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanolen).

Die lambda-max-Werte wurden an Hand der UV-Spektren von 200 nm bis 400 nm in den Maxima der chromatographischen Signale ermittelt.

### Anwendungsbeispiele

### Beispiel A

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Botrytis - Test (Bohne) / protektiv** | | |
|---|---|---|
| Wirkstoff | Aufwandmenge an Wirkstoff in ppm | Wirkungsgrad in % |
| Bekannt aus WO 03/070705 (Bsp. 10): | 100 | 52 |
| 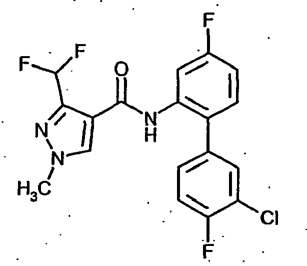 | | |
| Bekannt aus WO 03/070705 (Bsp. 12): | 100 | 87 |
| 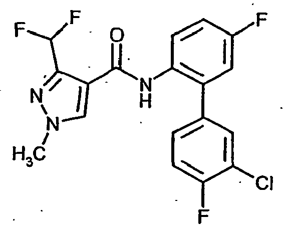 | | |
| Erfindungsgemäß: | 100 | 100 |
| 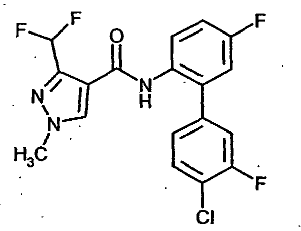 | | |

### Beispiel B

### Pyrenophora teres -Test (Gerste) / protektiv

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Pyrenophora teres -Test (Gerste) / protektiv** | | | |
|---|---|---|---|
| Wirkstoff | | Aufwandmenge an Wirkstoff in ppm | Wirkungsgrad in % |
| Bekannt aus WO 03/070705 (Bsp. 10): | | 1000 | 93 |
| 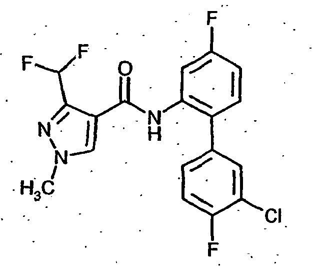 | | | |
| Lösungsmittel: | 25 Gew.-Teile N,N-Dimethylacetamid | | |
| Emulgator: | 0,6 Gew.-Teil Alkylarylpolyglykolether | | |
| Erfindungsgemäß: | | 1000 | 100 |
| 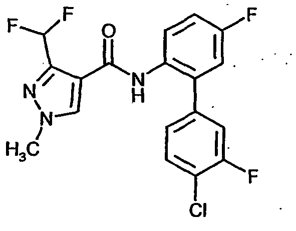 | | | |
| Lösungsmittel: | 50 Gew.-Teile N,N-Dimethylacetamid | | |
| Emulgator: | 1,0 Gew.-Teil Alkylarylpolyglykolether | | |

### Beispiel C

### Erysiphe-Test (Gerste) / protektiv

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Erysiphe-Test (Gerse) / protektiv** | | | |
|---|---|---|---|
| Wirkstoff | | Aufwandmenge an Wirkstoff in ppm | Wirkungsgrad in % |
| Bekannt aus WO 03/070705 (Bsp. 10): | | 1000 | 33 |
| 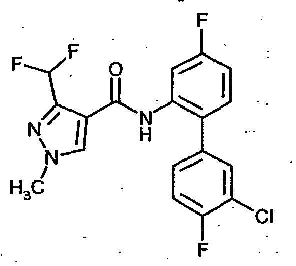 | | | |
| Lösungsmittel: | 25 Gew.-Teile N,N-Dimethylacetamid | | |
| Emulgator: | 0,6 Gew.-Teil Alkylarylpolyglykolether | | |
| Erfindungsgemäß: | | 1000 | 78 |
| 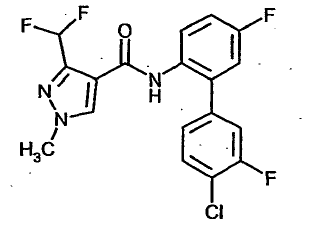 | | | |
| Lösungsmittel: | 50 Gew.-Teile N,N-Dimethylacetamid | | |
| Emulgator: | 1,0 Gew.-Teil Alkylarylpolyglykolether | | |

### Beispiel D

### Leptosphaeria nodorum-Test (Weizen) / protektiv

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

**Tabelle D**

| **Leptosphaeria nodorum-Test (Weizen) / protektiv** | | | |
|---|---|---|---|
| Wirkstoff | | Aufwandmenge an Wirkstoff in ppm | Wirkungsgrad in % |
| Bekannt aus WO 03/070705 (Bsp.10): | | 1000 | 0 |
| 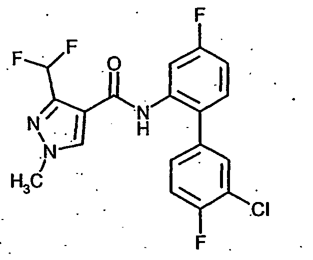 | | | |
| Lösungsmittel: | 25 Gew.-Teile N,N-Dimethylacetamid | | |
| Emulgator: | 0,6 Gew.-Teil Alkylarylpolyglykolether | | |
| Erfindungsgemäß: | | 1000 | 84 |
| 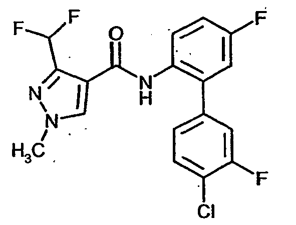 | | | |
| Lösungsmittel: | 50 Gew.-Teile N,N-Dimethylacetamid | | |
| Emulgator: | 1,0 Gew.-Teil Alkylarylpolyglykolether | | |

## Patentansprüche

1. Pyrazolylcarboxanilide der Formel (I) 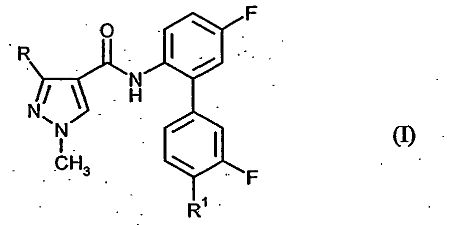 in welcher
R für Difluormethyl oder Trifluormethyl steht und
R¹ für Chlor oder Methyl steht.

2. Pyrazolylcarboxanilide der Formel (I) gemäß Anspruch 1, ausgewählt aus
N-(4'-Chlor-3',5-difluorbiphenyl-2-yl)-3-(difluormethyl)-1-methyl-1H-pyrazol-4-carboxamid
N-(4'-Chlor-3',5-difluorbiphenyl-2-yl)-1-methyl-3-(trifluormethyl)-1H-pyrazo1-4-carboxamid
N-(3',5-Difluor-4'-methylbiphenyl-2-yl)-1-methyl-3-(difluormethyl)-1H-pyrazol-4-carboxamid
N-(3',5-Difluor-4'-methylbiphenyl-2-yl)-1-methyl-3-(trifluormethyl)-1H-pyrazol4-carboxamid.

3. Verfahren zum Herstellen von Pyrazolylcarboxaniliden der Formel (I) gemäß Anspruch 1,
**dadurch gekennzeichnet, dass** man
a) Pyrazolylcarbonsäurehalogenide der Formel (II) 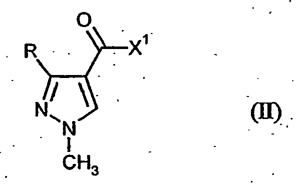 in welcher
R die in Anspruch 1 angegebenen Bedeutungen hat und
X¹ für Halogen steht,
mit Anilinderivaten der Formel (III) 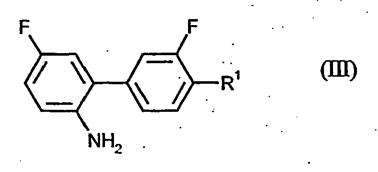 in welcher R¹ die in Anspruch 1 angegebenen Bedeutungen hat,
gegebenenfalls in Gegenwart eines Säurebindemittels und gegebenenfalls in Gegenwart eines Verdünnungsmittels umsetzt, oder
b) Halogenpyrazolylcarboxanilide der Formel (IV) 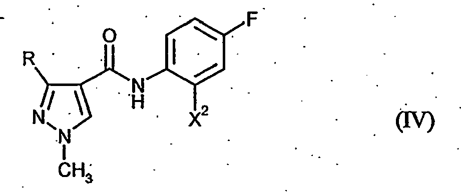 in welcher
R die in Anspruch 1 angegebenen Bedeutungen hat und
X² für Brom oder Iod steht,
mit Boronsäurederivaten der Formel (V) 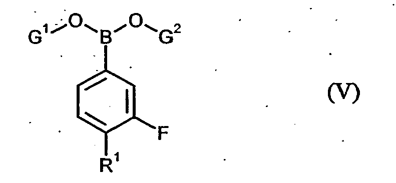 in welcher
R¹ die in Anspruch 1 angegebenen Bedeutungen hat und
G¹ und G² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt, oder
c) Halogenpyrazolylcarboxanilide der Formel (IV) 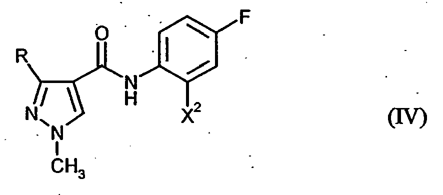 in welcher
R die in Anspruch 1 angegebenen Bedeutungen hat und
X² für Brom oder Iod steht,
in einer ersten Stufe mit einem Diboran-Derivat der Formel (VI) 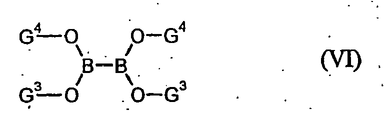 in welcher
G³ und G⁴ jeweils für Alkyl oder gemeinsam für Alkandiyl stehen,
in Gegenwart eines Katalysators, gegebenenfalls in Gegenwart eines Säurebindemittels und gegebenenfalls in Gegenwart eines Verdünnungsmittels umsetzt und ohne Aufarbeitung in einer zweiten Stufe mit Halogenbenzolderivaten der Formel (VII) 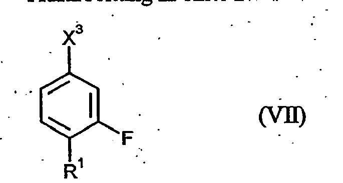 in welcher
R¹ die in Anspruch 1 angegebenen Bedeutungen hat und
X³ für Brom, Iod oder Trifluormethylsulfonyloxy steht,
in Gegenwart eines Katalysators, gegebenenfalls in Gegenwart eines Säurebindemittels und gegebenenfalls in Gegenwart eines Verdünnungsmittels umsetzt.

4. Mittel zum Bekämpfen unerwünschter Mikroorganismen, **gekennzeichnet durch** einen Gehalt an mindestens einem Pyrazolylcarboxanilid der Formel (I) gemäß Anspruch 1 neben Streckmitteln und/oder oberflächenaktiven Stoffen.

5. Verwendung von Pyrazolylcarboxaniliden der Formel (I) gemäß Anspruch 1 zum Bekämpfen unerwünschter Mikroorganismen ausgenommen am menschlichen oder tierischen Körper.

6. Verfahren zum Bekämpfen unerwünschter Mikroorganismen ausgenommen am menschlichen oder tierischen Körper, **dadurch gekennzeichnet, dass** man Pyrazolylcarboxanilide der Formel (I) gemäß Anspruch 1 auf die Mikroorganismen und/oder deren Lebensraum ausbringt.

7. Verfahren zum Herstellen von Mitteln zum Bekämpfen unerwünschter Mikroorganismen, **dadurch gekennzeichnet, dass** man Pyrazolylcarboxanilide der Formel (I) gemäß Anspruch 1 mit Streckmitteln und/oder oberflächenaktiven Stoffen verwischt.

8. Anilinderivaten der Formel (III) 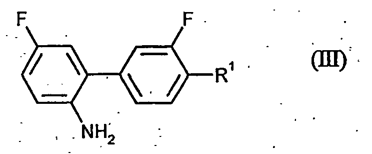 in welcher
R¹ für Chlor oder Methyl steht.

## Claims

1. Pyrazolylcarboxanilides of the formula (I) 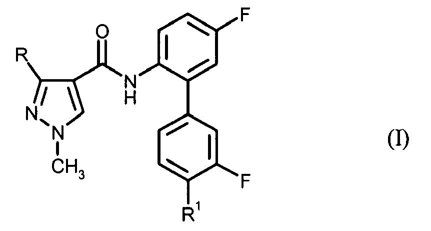 in which
R represents difluoromethyl or trifluoromethyl and
R¹ represents chlorine or methyl.

2. Pyrazolylcarboxanilides of the formula (I) according to Claim 1, selected from the group consisting of
N-(4'-chloro-3',5-difluorobiphenyl-2-yl)-3-(difluoromethyl)-1-methyl-1H-pyrazole-4-carboxamide
N-(4'-chloro-3',5-difluorobiphenyl-2-yl)-1-methyl-3-(trifluoromethyl)-1H-pyrazole-4-carboxamide
N-(3',5-difluoro-4'-methylbiphenyl-2-yl)-1-methyl-3-(difluoromethyl)-1H-pyrazole-4-carboxamide
N-(3',5-difluoro-4'-methylbiphenyl-2-yl)-1-methyl-3-(trifluoromethyl)-1H-pyrazole-4-carboxamide.

3. Process for the preparation of pyrazolylcarboxanilides of the formula (I) according to Claim 1, **characterized in that**
a) pyrazolylcarboxylic acid halides of the formula (II) 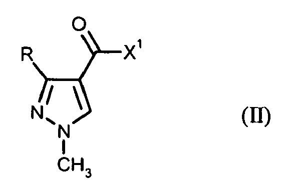 in which
R has the meanings given in Claim 1 and
X¹ represents halogen
are reacted with aniline derivatives of the formula (III) 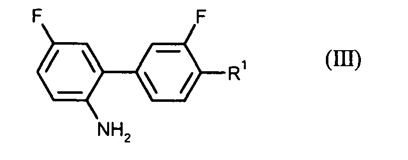 in which R¹ has the meanings given in Claim 1,
if appropriate in the presence of an acid binder and if appropriate in the presence of a diluent, or
b) halopyrazolylcarboxanilides of the formula (IV) 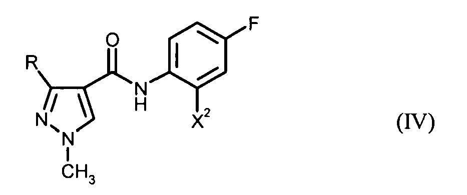 in which
R has the meanings given in Claim 1 and
X² represents bromine or iodine
are reacted with boronic acid derivatives of the formula (V) 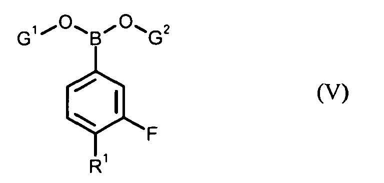 in which
R¹ has the meanings given in Claim 1 and
G¹ and G² represent in each case hydrogen or together represent tetramethylethylene in the presence of a catalyst, if appropriate in the presence of an acid binder and if appropriate in the presence of a diluent, or
c) halopyrazolylcarboxanilides of the formula (IV) 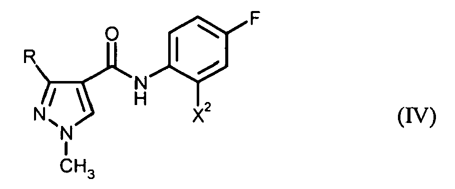 in which
R has the meanings given in Claim 1 and
X² represents bromine or iodine
are reacted, in a first step, with a diborane derivative of the formula (VI) 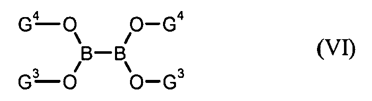 in which
G³ and G⁴ represent in each case alkyl or together represent alkanediyl
in the presence of a catalyst, if appropriate in the presence of an acid binder and if appropriate in the presence of a diluent and, without work-up, in a second step with halobenzene derivatives of the formula (VII) 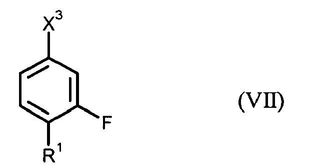 in which
R¹ has the meanings given in Claim I and
X³ represents bromine, iodine or trifluoromethylsulphonyloxy
in the presence of a catalyst, if appropriate in the presence of an acid binder and if appropriate in the presence of a diluent.

4. Composition for controlling undesired microorganisms, **characterized by** a content of at least one pyrazolylcarboxanilide of the formula (I) according to Claim 1 in addition to extenders and/or surface-active substances.

5. Use of pyrazolylcarboxanilides of the formula (I) according to Claim 1 for controlling undesired microorganisms except from on the human or animal body.

6. Method of controlling undesired microorganisms except from on the human or animal body, **characterized in that** pyrazolylcarboxanilides of the formula (I) according to Claim 1 are applied to the microorganisms and/or their environment.

7. Process for the preparation of compositions for controlling undesired microorganisms, **characterized in that** pyrazolylcarboxanilides of the formula (I) according to Claim 1 are mixed with extenders and/or surface-active substances.

8. Aniline derivatives of the formula (III) 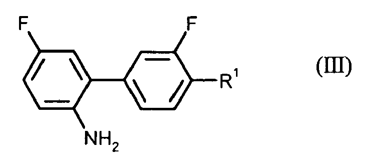 in which
R¹ represents chlorine or methyl.

## Revendications

1. Pyrazolylcarboxanilides de la formule (I) : 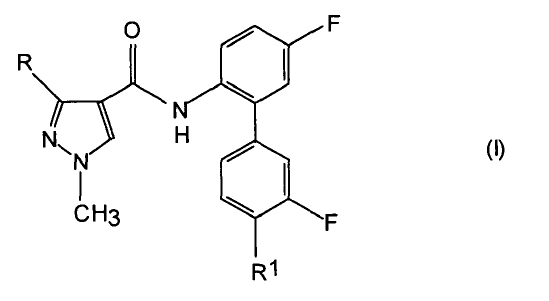 dans laquelle :
R représente le radical difluorométhyle ou trifluorométhyle, et
R' représente l'atome de chlore ou le radical méthyle.

2. Pyrazolylcarboxanilides de la formule (I) selon la revendication 1, choisis parmi :
le N-(4'-chloro-3',5-difluorobiphényl-2-yl)-3-(difluorométhyl)-1-méthyl-1H-pyrazol-4-carboxamide,
le N-(4'-chloro-3',5-difluorobiphényl-2-yl)-1-méthyl-3-(trifluorométhyl)-1H-pyrazol-4-carboxamide,
le N-(3',5-difluoro-4'-méthylbiphényl-2-yl)-1-méthyl-3-(difluorométhyl)-1H-pyrazol-4-carboxamide,
le N-(3',5-difluoro-4'-méthylbiphényl-2-yl)-1-méthyl-3-(trifluorométhyl)-1H-pyrazol-4-carboxamide.

3. Procédé de préparation des pyrazolylcarboxanilides de la formule (I) selon la revendication 1, **caractérisé en ce que** :
a) on fait réagir des halogénures d'acide pyrazolylcarboxylique de la formule (II) : 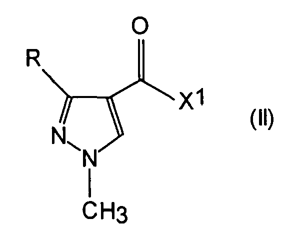 dans laquelle :
R a les significations indiquées à la revendication 1, et
X¹ représente un atome d'halogène,
avec des dérivés d'aniline de la formule (III) : 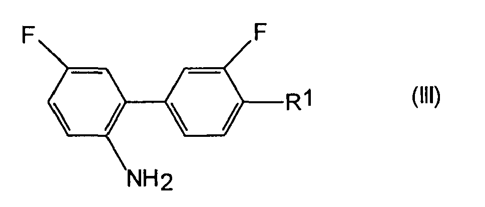 dans laquelle R¹ a les significations indiquées à la revendication 1,
le cas échéant en présence d'un agent liant les acides et le cas échéant, en présence d'un agent de dilution, ou
b) on fait réagir les halogénopyrazolyl-carboxanilide de la formule (IV) : 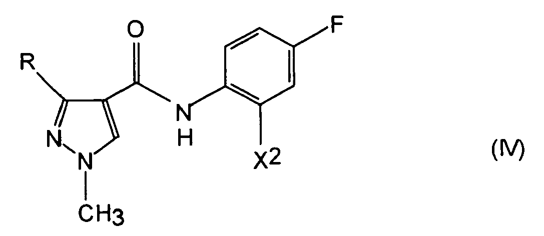 dans laquelle :
R a les significations indiquées à la revendication 1, et
X² représente l'atome de brome ou d'iode,
avec des dérivés d'acide boronique de la formule (V) : 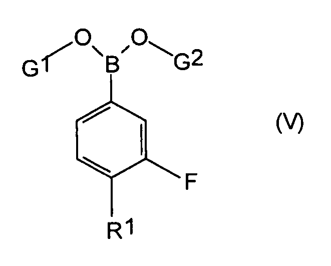 dans laquelle :
R¹ a les significations indiquées à la revendication 1, et
G¹ et G² représentent chaque fois, l'atome d'hydrogène ou ensemble, tétraméthyléthylène,
en présence d'un catalyseur, le cas échéant en présence d'un agent liant les acides et le cas échéant, en présence d'un agent de dilution, ou
c) on fait réagir les halogénopyrazolyl-carboxanilide de la formule (IV) : 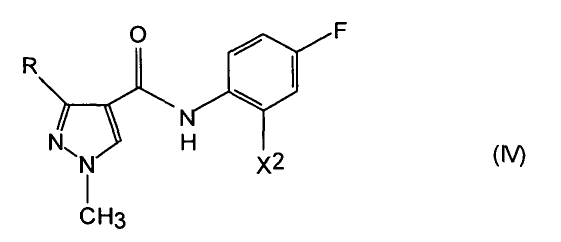 dans laquelle :
R a les significations indiquées à la revendication 1, et
X² représente l'atome de brome ou d'iode,
dans une première étape, avec des dérivés diboranes de la formule (VI) : 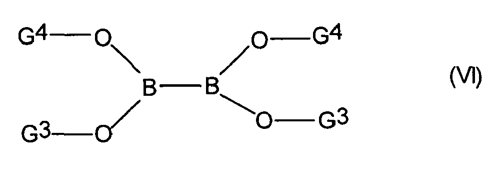 dans laquelle :
G³ et G⁴ représente chaque fois, un radical alcoyle ou ensemble, un radical alcanediyle,
en présence d'un catalyseur, le cas échéant en présence d'un agent liant les acides et le cas échéant, en présence d'un agent de dilution, et sans traitement dans une deuxième étape, avec des dérivés halogénobenzènes de la formule (VII) : 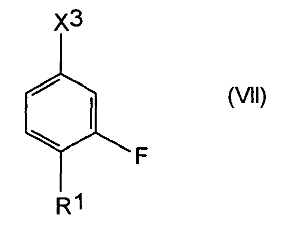 dans laquelle :
R¹ a les significations indiquées à la revendication 1, et
X³ représente l'atome de brome, d'iode ou le radical trifluorométhylsulfonyloxy,
en présence d'un catalyseur, le cas échéant en présence d'un agent liant les acides et le cas échéant, en présence d'un agent de dilution.

4. Agent pour lutter contre les microorganismes non désirés, **caractérisé par** une teneur en au moins un pyrazolylcarboxanilide de la formule (I) selon la revendication 1, avec des diluants et/ou des agents tensioactifs.

5. Utilisation des pyrazolylcarboxanilides de la formule (I) selon la revendication 1, pour lutter contre des microorganismes non désirés, à l'exclusion des corps humains ou animaux.

6. Procédé pour lutter contre des microorganismes non désirés, à l'exclusion des corps humains ou animaux, **caractérisé en ce que** l'on applique les pyrazolylcarboxanilides de la formule (I) selon la revendication 1, sur les microorganismes et/ou leur biotope.

7. Procédé de préparation d'agents pour lutter contre des microorganismes non désirés, à l'exclusion des corps humains ou animaux, **caractérisé en ce que** l'on mélange les pyrazolylcarboxanilides de la formule (I) selon la revendication 1, avec des diluants et/ou des agents tensioactifs.

8. Dérivés d'aniline de la formule (III) : 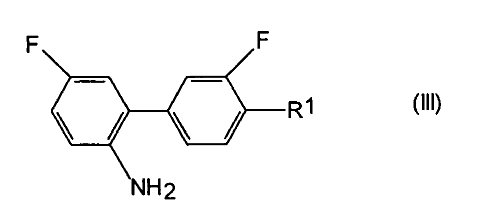 dans laquelle R¹ représente l'atome de chlore ou le radical méthyle.
